# EUROPÄISCHE PATENTSCHRIFT

(11) **EP 2 994 452 B1**
(45) Veröffentlichungstag und Bekanntmachung des Hinweises auf die Patenterteilung: **20.06.2018**
(21) Anmeldenummer: 14720100.8
(22) Anmeldetag: 25.04.2014
(51) Int. Cl.: C07C 201/08, C07C 205/02, B01J 19/00

(54) **VERFAHREN ZUR HERSTELLUNG VON NITROALKANEN IN EINEM MIKROSTRUKTURIERTEN REAKTOR**
METHOD FOR THE PRODUCTION OF NITROALKANES IN A MICROSTRUCTURED REACTOR
PROCÉDÉ DE FABRICATION DE NITROALCANES DANS UN RÉACTEUR MICROSTRUCTURÉ

(30) Priorität: 06.05.2013 EP 13166644
(43) Veröffentlichungstag der Anmeldung: 16.03.2016
(73) Patentinhaber: BASF SE, 67056 Ludwigshafen am Rhein (DE)
(72) Erfinder: BÖHLING, Ralf, 64653 Lorsch (DE); HAYER, Michael, 67227 Frankenthal (DE); REHFINGER, Alwin, 67112 Mutterstadt (DE); SCHELPER, Michael, 69126 Heidelberg (DE); MELDER, Johann-Peter, 67459 Böhl-Iggelheim (DE); ERNST, Martin, 69121 Heidelberg (DE); TELES, Joaquim Henrique, 67165 Waldsee (DE)
(74) Vertreter: BASF IP Association
(86) Internationale Anmeldenummer: PCT/EP2014/058437
(87) Internationale Veröffentlichungsnummer: WO 2014/180678

(56) Entgegenhaltungen:
- EP-A1- 1 932 821
- EP-A1- 2 048 129
- GB-A- 586 203

## Beschreibung

Die Erfindung betrifft die Herstellung von Nitroalkanen durch Umsetzung mindestens eines Alkans mit mindestens einem Nitrierungsmittel in der Gasphase.

Nitroalkane finden als industrielle Lösemittel Verwendung und stellen wichtige Synthesebausteine für komplexere Moleküle dar. Die vier aus industrieller Sicht wichtigsten Nitroalkane Nitromethan, Nitroethan, 1-Nitropropan und 2-Nitropropan werden üblicherweise durch Hochtemperatur-Dampfphasen-Nitrierung von Propan hergestellt (Ullmann's Encyclopedia of Industrial Chemistry, "Nitro Compounds, Aliphatic", Kapitel 3 "Production", Wiley-VCH Verlag, Weinheim, 2005). Als Nitrierungsmittel der radikalisch verlaufenden Reaktion wird Salpetersäure eingesetzt, wobei das NO₂-Radikal die eigentlich aktive Spezies darstellt. Die Umsetzung erfolgt bei einer Temperatur von 350°C bis 450°C und einem Druck von 8 bis 12 bar. Die Temperaturkontrolle der stark exothermen Reaktion wird dabei auf unterschiedlichen Wegen erzielt, beispielsweise durch eine Fahrweise mit Propanüberschuss, dem Einsprühen und Verdampfen flüssiger Salpetersäure oder der Verkürzung der Verweilzeit im Reaktor. Der Salpetersäureumsatz zu den Zielprodukten liegt unter 50%. Der überwiegende Teil reagiert zu NOₓ und N₂, wobei ersteres zurückgewonnen wird. Bei Rückführung von überschüssigem Propan können 60% bis 80% des Propans zu Nitroalkanen umgewandelt werden.

Die Patentschrift GB 586,203 offenbart ein Verfahren zur Herstellung von Nitroparaffinen durch Umsetzung von Paraffinen mit einem Nitrierungsmittel wie Stickstoffdioxid oder Salpetersäure in der Gasphase. Das Reaktionsgefäß ist im Inneren mit einem Futter ausgekleidet, dessen innere Oberfläche aus einem glasartigen, keramischen oder feuerfesten Material besteht. Als Beispiel wird die Umsetzung von Propan mit Stickstoffdioxid zu Nitropropan bei einer Temperatur von 320°C bis 350°C und einem Druck von 5,2 bar beschrieben, wobei die Reaktion in einem Reaktor durchgeführt wird, dessen Reaktionskanäle aus Bor-Silikat-Glas bestehen.

In der Patentschrift US 4,626,607 wird ein Verfahren zur Herstellung von Nitromethan beschrieben. Dabei wird Methan in einer homogenen Gasphasenreaktion mit Salpetersäure oder Stickstoffdioxid bei Drücken von 1 bis 35 bar und Temperaturen von 270°C bis 600°C in Gegenwart eines Aktivators umgesetzt. Als Aktivator kommen Halogene wie Chlor und Brom oder Derivate davon zum Einsatz.

Die EP 0174600 B1 beschreibt ein Verfahren zur Herstellung von Nitroalkanen und nitroaromatischen Verbindungen aus einem olefinisch ungesättigten Kohlenwasserstoff, beispielsweise ein Alken wie Propylen oder Buten. Das Olefin wird in einer Reaktionszone mit Stickstoffdioxid oder Salpetersäure bei einem Druck von 2 bis 20 bar und einer Temperatur von 100°C bis 500°C kontaktiert.

Auch zweiphasige Prozesse sind bekannt. So beschreibt die WO 2009/129099 A1 ein Herstellverfahren für Nitropropane wie 2-Nitropropan und 2,2-Dinitropropan in einem Trickle-Bed-Reaktor. Dabei strömt gasförmiges Propan im Gegenstrom zu einer im Reaktor senkrecht herablaufenden wässrigen Salpetersäurelösung bei einem Druck von 68 bis 109 bar und einer Temperatur von 215°C bis 325°C.

Das Dokument EP 1 932 821 A1 offenbart die Oxidation von flüssigem Cyclohexan mit sauerstoffhaltigen Gasen bei erhöhter Temperatur und unter erhöhtem Druck in einer mikrostrukturierten Reaktionszone mit parallelen Kanälen.

In dem Dokumente EP 2 048 129 A1 wird die Nitrierung von Alkoholen zu Nitryloxy-Verbindungen in Mikroreaktoren mit mehreren Einspeisestellen beschrieben.

Den bekannten Verfahren ist gemein, dass aufgrund des Gefährdungspotenzials der verwendeten Substanzen ein hoher sicherheitstechnischer Aufwand erforderlich ist, was aufwändige und teure Apparaturen erfordert. Zudem ist die Selektivität hinsichtlich des gewünschten Nitroalkans in den meisten Fällen nicht zufriedenstellend.

Es stellte sich die Aufgabe, ein Verfahren bereitzustellen, das eine hohe Selektivität des gewünschten Nitroalkans aufweist. Unter der Selektivität wird hier und im Folgenden der Quotient aus der Molmenge an gebildetem Nitroalkan und der Molmenge des umgesetzten Alkans verstanden. Ferner soll das Verfahren kostengünstig in einer Apparatur durchgeführt werden können, die den hohen sicherheitstechnischen Anforderungen aufgrund der beteiligten Stoffe genügt.

Diese Aufgabe wird durch den Gegenstand der Erfindung gelöst, wie er in Anspruch 1 wiedergegeben ist. Weitere vorteilhafte Ausführungsformen der Erfindung finden sich in den abhängigen Ansprüchen.

Erfindungsgemäß wird mindestens ein Alkan mit mindestens einem Nitrierungsmittel in einer mikrostrukturierten Reaktionszone in der Gasphase umgesetzt. Das mindestens eine Alkan kann als Reinstoff oder als Gemisch mit anderen Substanzen vorliegen, beispielsweise als Gemisch mit anderen Alkanen. Das mindestens eine Alkan sowie das mindestens eine Nitrierungsmittel werden gasförmig unter einem Druck von 1 bar bis 20 bar durch die Reaktionszone gefördert und bei einer Temperatur von 150°C bis 650°C, bevorzugt von 200°C bis 350°C, besonders bevorzugt von 250°C bis 300°C umgesetzt. Die Reaktionsprodukte werden nach der Reaktionszone gekühlt und zur weiteren Verwendung ausgetragen.

Bevorzugt wird der Druck in der Reaktionszone möglichst hoch gewählt. Allerdings sollte dabei gewährleistet sein, dass die Einsatzstoffe und die Reaktionsprodukte in der Reaktionszone noch dampfförmig vorliegen. Zudem ist sicherzustellen, dass im Falle einer Detonation der sich maximal einstellende Detonationsdruck in der mikrostrukturierten Reaktionszone noch beherrschbar ist.

Vorzugsweise handelt es sich bei den Alkanen um verzweigte und/oder nicht verzweigte Alkane mit einem bis zwanzig Kohlenstoffatomen.

Geeignete Nitrierungsmittel umfassen Stickstoffoxide (NOₓ), insbesondere Stickstoffmonoxid und Stickstoffdioxid. Als besonders geeignet haben sich Gemische aus Stickstoffoxiden und Sauerstoff erwiesen, insbesondere Gemische aus Stickstoffmonoxid und Sauerstoff. In einer bevorzugten Variante werden Stickstoffmonoxid und Sauerstoff gemischt, wobei Stickstoffdioxid entsteht, bevor dieses Nitrierungsmittelgemisch mit dem Alkan in Kontakt gebracht wird.

Unter einer mikrostrukturierten Reaktionszone im Sinne der Erfindung wird eine Zone eines Reaktors verstanden, die mit parallelen Kanälen mit hydraulischen Durchmessern von weniger als 2,5 mm, bevorzugt weniger als 1,6 mm, und einer spezifischen inneren Oberfläche von mehr als 1600 m²/m³, vorzugsweise mehr als 2500 m²/m³ versehen ist. Der hydraulische Durchmesser (dₕ) ist nach allgemeinem Verständnis definiert als das Vierfache des Quotienten aus Durchflussquerschnitt (A_{d}) und benetztem Umfang (U_{d}) eines Rohres oder Kanals mit nicht kreisförmigem Querschnitt, sodass gilt dₕ = 4·A_{d} / U_{d}. Die spezifische innere Oberfläche (Aₛᵢ) ist gleich dem Quotient aus benetzter Oberfläche (A_{b}) und Volumen (V) des Rohres oder Kanals, also Aₛᵢ = A_{b}/ V. Für einen rechteckigen Kanalquerschnitt mit der Breite b und der Höhe h beispielsweise ergibt sich somit unter der Annahme, dass sich der Querschnitt über die Länge des Kanals nicht ändert, ein hydraulischer Durchmesser von dₕ = 2·b·h / (b+h) und, unter der weiteren Annahme, dass die gesamte innere Oberfläche des Kanals benetzt ist, eine spezifische innere Oberfläche von Aₛᵢ = 2·(b+h) / (b·h). Im Falle eines kreisrunden Kanalquerschnitts mit dem Durchmesser "d" ergeben sich unter denselben Bedingungen ein hydraulischer Durchmesser von dₕ = d und eine spezifische innere Oberfläche von Aₛᵢ = 4 / d.

Die minimale Abmessung in jeder Richtung eines Querschnitts durch einen Kanal beträgt vorzugsweise 0,05 mm, besonders bevorzugt 0,1 mm, insbesondere 0,2 mm. Dieser Wert wird bestimmt von der Fertigungstoleranz und dem Fertigungsaufwand bei der Herstellung der mikrostrukturierten Reaktionszone sowie der Anfälligkeit gegenüber Verstopfungen. Die Kanäle in der Reaktionszone werden im Folgenden auch als Reaktionskanäle bezeichnet. Eine solche Reaktionszone kann aus modular zusammengesetzten Bauteilen gebildet werden, wobei die einzelnen Bauteile an jeweils übereinstimmenden Stellen Bohrungen oder Vertiefungen haben, die in Gebrauchsstellung Kanäle bilden, durch die Stoffströme für die Reaktion oder den Wärmetransport geführt werden können. Mikrostrukturierte Reaktoren zeichnen sich durch eine sehr gute Wärmeabfuhr verbunden mit einem sehr schnellen Stofftransport aus. Die Wandstärken zwischen den Kanälen sind so zu wählen, dass eine Eigensicherheit bei potenziell explosiven Stoffgemischen gewährleistet ist.

In einer bevorzugten Ausführungsform der Erfindung wird die Nitrierung in Gegenwart einer inerten oder passivierten inneren Oberfläche der mikrostrukturierten Reaktionszone durchgeführt. Zur Herstellung einer inerten oder passivierten inneren Oberfläche können die Reaktionskanäle mit einer Beschichtung versehen sein. Besonders bevorzugt ist die innere Oberfläche der mikrostrukturierten Reaktionszone zumindest teilweise, insbesondere vollständig, mit einer Siliziumbeschichtung versehen.

Eine inerte oder passivierte innere Oberfläche der Reaktionskanäle trägt dazu bei, dass unerwünschte Nebenreaktionen an den Wänden der Reaktionskanäle verringert oder gänzlich unterdrückt werden, was sich vorteilhaft auf die Selektivität und Ausbeute des gewünschten Produktes auswirkt.

In einer alternativen, bevorzugten Ausgestaltung der Erfindung werden die Materialien zur Herstellung der mikrostrukturierten Reaktionszone so gewählt, dass sie dieselben positiven Eigenschaften wie die oben beschriebene Beschichtung aufweisen. Besonders bevorzugt sind die Innenwände der Reaktionskanäle aus einem inerten Material gefertigt, insbesondere aus Silizium, Siliziumcarbid oder einem Glas, das bezüglich des Inertverhaltens vergleichbare Eigenschaften wie Quarzglas oder Borosilikatglas aufweist. Besonders vorteilhaft ist die gesamte mikrostrukturierte Reaktionszone aus einem derartigen inerten Material gefertigt.

Ein wesentlicher Vorteil der mikrostrukturierten Reaktionszone gegenüber anderen Reaktorkonzepten ist die Möglichkeit, den Reaktor eigensicher auszulegen. Besonders bevorzugt wird die Wandstärke zwischen zwei Hohlräumen an jeder Stelle in der mikrostrukturierten Reaktionszone so gewählt, dass die Eigensicherheit gegen detonative Explosionen gegeben ist.
Bevorzugt wird vor dem Einbringen in die Reaktionskanäle das Alkan oder das Gemisch von Alkanen mit dem einen oder den mehreren Nitrierungsmitteln gemischt, sodass ein homogenes gasförmiges Reaktionsgemisch entsteht. Liegt ein Einsatzstoff in flüssiger Form vor, wird er vorzugsweise zunächst verdampft, bevor er mit den übrigen Einsatzstoffen vermischt wird. Geeignete mikrostrukturierte Mischorgane sind dem Fachmann bekannt, ebenso geeignete Verteilerstrukturen, um das Reaktionsgemisch gleichmäßig auf die Reaktionskanäle zu verteilen. Das Vermischen der Einsatzstoffe erfolgt vorteilhaft unterhalb der Reaktionstemperatur bei einem Druck, bei dem die beteiligten Stoffe dampfförmig vorliegen. Dadurch wird ein unerwünschtes Abreagieren des Gemisches außerhalb der Reaktionszone vermieden.
Erfindungsgemäß wird zumindest ein Teil der Einsatzstoffe erst in der Reaktionszone miteinander vermischt. Das mindestens eine Nitrierungsmittel wird über zwei bis zehn, insbesondere vier bis sechs Einspeisestellen entlang der Reaktionszone verteilt zugeführt.
In einer vorteilhaften Ausgestaltung, bei der ein Gemisch aus Stickstoffoxiden und Sauerstoff als Nitrierungsmittel eingesetzt wird, hat es sich als vorteilhaft erwiesen, ein stickstoffoxidreiches Teilgemisch am Reaktoreingang zuzuführen und über die Einspeisestellen entlang der Reaktionszone ein sauerstoffreiches Teilgemisch zuzuführen. Besonders vorteilhaft wird über die Einspeisestellen entlang der Reaktionszone ein überwiegend Sauerstoff enthaltendes Teilgemisch zugeführt, ganz besonders bevorzugt wird elementarer Sauerstoff zugeführt. Stickstoffdioxid steht in einem Gleichgewicht mit Stickstoffmonoxid und Sauerstoff. Stickstoffdioxid zerfällt unter den typischen Reaktionsbedingungen zu Stickstoffmonoxid und Sauerstoff. Es hat sich als vorteilhaft erwiesen, diese sogenannte Dissoziation des Stickstoffmonoxids auf maximal 15% zu begrenzen. Der Dissoziationsgrad lässt sich dadurch beeinflussen, dass Druck und Temperatur in der Reaktionszone adäquat gewählt werden. Ferner bewirkt eine Zwischeneinspeisung eines sauerstoffreichen Teilgemisches eine Verringerung des Dissoziationsgrades. Das molare Verhältnis von Alkanen zu Nitrierungsmittel beträgt bevorzugt von 1:10 bis 10:1, besonders bevorzugt von 1:5 bis 5:1. Die Umsetzung von Alkanen mit dem oder den Nitrierungsmitteln erfolgt bevorzugt bei einer Verweilzeit in den Reaktionskanälen von 1 s bis 600 s, insbesondere von 20 s bis 200 s.
In einer weiteren Ausführungsform wird die Nitrierung in Gegenwart eines heterogenen Katalysators durchgeführt. Vorzugsweise ist der Katalysator in fester Form, beispielsweise als Schüttung, in den Reaktionskanälen vorhanden. Der Katalysator kann auch in Form einer Beschichtung auf den Innenwänden der Reaktionskanäle angebracht sein. In diesem Fall wirkt die katalytische Beschichtung im Hinblick auf unerwünschte Nebenreaktionen wie eine inerte oder passivierte innere Oberfläche der mikrostrukturierten Reaktionszone.

In einer weiteren erfindungsgemäßen Ausführungsform werden dem Reaktionsgemisch zusätzlich eine oder mehrere inerte Substanzen zugeführt, vorzugsweise Stickstoff oder Wasser. Bevorzugt werden die inerten Substanzen dem Reaktionsgemisch vor Eintritt in die Reaktionszone zugeführt. In einer weiteren bevorzugten Variante werden die inerten Substanzen innerhalb der Reaktionszone dem Reaktionsgemisch zugeführt.

In einer erfindungsgemäßen Ausführungsform werden die Teilströme aus den Reaktionskanälen nach dem Austritt aus der Reaktionszone zusammengeführt und das Reaktionsprodukt ausgetragen. Die bei einer Abkühlung des Reaktionsproduktes entstehenden Gas- und Flüssigphasen können voneinander getrennt und anschließend mittels bekannter Verfahren wie der fraktionierenden Destillation aufbereitet werden. Nicht umgesetzte Einsatzstoffe können zur mikrostrukturierten Reaktionszone zurückgeführt werden.

In einer vorteilhaften Weiterbildung der Erfindung werden die Reaktionsprodukte nach Austritt aus der Reaktionszone in einer weiteren mikrostrukturierten Zone um mindestens 10°C, bevorzugt um mindestens 30°C abgekühlt. Diese Zone wird im Folgenden auch als Quenchzone bezeichnet. Die Temperaturen in der Reaktionszone und der Quenchzone sind vorzugsweise getrennt voneinander regelbar. Durch das schnelle Abkühlen der Reaktionsprodukte nach dem Austritt aus der Reaktionszone wird eine mögliche Weiterreaktion außerhalb der Reaktionszone vermindert oder vollständig unterbunden, was sich vorteilhaft auf die gewünschte Selektivität auswirkt.

In einer erfindungsgemäßen Weiterbildung werden die Reaktionsprodukte nach dem Austritt aus der Reaktionszone oder der Abkühlzone einer weiteren Synthesestufe zugeführt. Dort können die Nitroalkane beispielsweise mit einem Aldehyd oder Keton (Henry-Reaktion) oder in einer Michael-Addition (z.B. Nitrile oder Ester) umgesetzt werden.

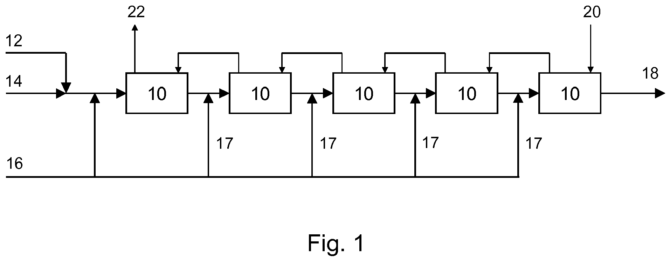

Fig. 1 zeigt schematisch ein bevorzugtes Reaktorkonzept zur Durchführung des erfindungsgemäßen Verfahrens. Das Reaktorkonzept sieht eine Reaktorkaskade aus mehreren Reaktormodulen 10 vor, die im dargestellten Beispiel fünfstufig ausgeführt ist. Vor dem Eintritt in das erste Reaktormodul 10 wird über eine erste Zuleitung 12 das gasförmige Alkan und über eine zweite Zuleitung 14 das gasförmige Nitrierungsmittel, vorzugsweise Stickstoffmonoxid, zusammengeführt und vermischt. Nachfolgend wird über einen Sauerstoffzulauf 16 dem Gemisch Sauerstoff zugeführt, und das resultierende Reaktionsgemisch in das erste Reaktormodul geleitet. Zwischen den einzelnen Reaktormodulen 10 ist jeweils eine Sauerstoffzwischeneinspeisung 17 vorgesehen, die im Hinblick auf den Mengenstrom bevorzugt individuell regelbar ist. Zur Abfuhr der bei der Reaktion entstehenden Wärme werden die Reaktormodule 10 von einem Kühlmedium durchströmt. In dem dargestellten Beispiel strömt das Kühlmedium im Gegenstrom zum Reaktionsgemisch durch die Reaktorkaskade. Das Kühlmedium wird über einen Kühlmediumzulauf 20 dem auf die Kaskade bezogen hintersten Reaktormodul zugeführt, durchströmt die Module der Reihe nach von hinten nach vorne und verlässt das vorderste Modul durch einen Kühlmediumauslass 22. Je nach Anforderung an die abzuführende Wärme sind selbstverständlich auch andere Kühlkonzepte realisierbar, neben der dargestellten Reihenschaltung im Gegenstrom beispielsweise auch Varianten im Gleichstrom, Kreuzstrom oder Parallelschaltungen. Entsprechende Verschaltungen und geeignete Kühlmedien sind dem Fachmann bekannt.

Gegenüber dem Stand der Technik, bei dem für hohe Raumzeitausbeuten erhebliche Alkanüberschüsse erforderlich sind, kann nach dem erfindungsgemäßen Verfahren ohne Alkanüberschüsse eine hohe Selektivität von bis zu 63% Nitroalkanen gesamt bei Umsätzen von bis zu 90% an Alkanen gesamt erzielt werden. Die mikrostrukturierte Reaktionszone ist störunanfällig und kann besonders kleinbauend ausgeführt sein. Zusätzliche Vorkehrungen für die gleichmäßige Aufteilung des Komponentengemischs in die einzelnen Kanäle sind nicht erforderlich. Die Synthese kann in der mikrostrukturierten Reaktionszone eigensicher ausgeführt werden.

In einer bevorzugten Ausgestaltung des erfindungsgemäßen Verfahrens wird Propan eingesetzt, um hochselektiv 2-Nitropropan herzustellen. Bevorzugte Nitrierungsmittel sind in diesem Fall Stickstoffdioxid sowie eine Mischung aus Stickstoffmonoxid und Sauerstoff. Bei der Umsetzung von Propan mit Stickstoffdioxid oder Stickstoffmonoxid beträgt das Verhältnis der Einsatzstoffe vorzugsweise von 0,8 bis 1,5 mol NOₓ / mol Propan.

In einer weiteren bevorzugten Ausgestaltung des erfindungsgemäßen Verfahrens wird Isooktan mit einer Mischung aus Stickstoffmonoxid und Sauerstoff zu Nitro-Isooktan umgesetzt.

Gegenüber etablierten Methoden zur Herstellung von Nitroalkanen weist das erfindungsgemäße Verfahren die Vorteile auf, dass aufgrund der mikrostrukturierten Reaktionszone eine zufriedenstellende und zuverlässige Temperaturkontrolle und damit ein eigensicherer Betrieb möglich sind. Das Gefährdungspotenzial ist dadurch erheblich verringert. Die erforderlichen Apparaturen sind einfach im Aufbau, kostengünstig umsetzbar und modulartig skalierbar.

Das erfindungsgemäße Verfahren wird durch die nachfolgenden Beispiele näher erläutert.

### Propan-Nitrierung

Der Versuchsaufbau zur Synthese von Nitropropan entspricht im Wesentlichen dem in Fig. 1 dargestellten Reaktorkonzept, die Zuführung der Einsatzstoffe ist jedoch leicht abgewandelt. Stickstoffmonoxid (NO) als Nitrierungsmittel und Sauerstoff werden separat aus Gasflaschen gasförmig mengengeregelt einem ersten Mischer zugeführt, wobei bereits Stickstoffdioxid (NO₂) entsteht. Das Gasgemisch wird einem zweiten Mischer zugeführt. Propan wird zunächst flüssig aus einer mit He-Druck überlagerten Stahlflasche flussgeregelt durch eine auf 100°C eingestellte Thermostatzone in eine Kapillare geleitet. Dort verdampft das Propan und wird in den zweiten Mischer geleitet. Vom zweiten Mischer wird das Reaktionsgemisch in ein erstes Reaktormodul überführt.

Als Reaktor wurde ein 1/8"-Kapillarrohr (Innendurchmesser 1,4 mm) gewählt, das in unterschiedlichen Längen bis zu 60 m zur Verfügung steht und zylindrisch aufgewickelt ist. Es gewährleistet einen ausreichenden Wärmeübergang und befindet sich in einem rohrförmigen Heißluftofen, der fönbeheizt auf Temperaturen bis zu 400°C geregelt werden kann. Dazu werden die Lufttemperatur sowie der Durchsatz an Heißluft entsprechend eingestellt. Außerhalb des Heißluftofens ist zusätzlich eine Schutzheizung vorgesehen, um Wärmeverluste ausgleichen und dadurch eine gleichmäßige Lufttemperatur im Ofen gewährleisten zu können. Das Kapillarrohr ist zur Inertisierung auf seiner Innenfläche mit Silizium beschichtet. Für Vergleichsversuche wurde ein nicht beschichtetes Kapillarrohr verwendet.

Für die Einspeisung von Sauerstoff sind außerhalb des Ofengehäuses mehrere Einspeisestellen vorgesehen, die als T-Stücke realisiert sind. An diesen Stellen ist die Reaktionskapillare aus dem Ofenraum herausgeführt, mit dem T-Stück verbunden und wieder in den Ofenraum zurückgeführt. Dabei ragt die Reaktionskapillare nicht mehr als 5 cm aus dem Ofenraum heraus. Der Sauerstoff wird aus einer Stahlflasche mengengeregelt zu den Einspeisestellen geführt.

Der Eingangsdruck der Mischer sowie des Reaktors werden über Sicherheitsventile in den Zuleitungen auf den definierten Maximaldruck der Synthese, in diesem Fall 15 bar, abgesichert.

Das den Kapillarreaktor verlassende Produktgemisch wird in eine Zone geleitet, die auf ca. 200°C temperiert ist, um eine Kondensation zu verhindern. Das Gasgemisch wird druckgeregelt auf Umgebungsdruck entspannt und über ein Probenahmeventil einem Gaschromatograph zur Online-Analyse zugeführt. Von dort gelangt der Gasstrom in einen Quenchbehälter, in dem der Gasstrom in eine Quenchflüssigkeit geleitet wird, wo er zumindest teilweise auskondensiert. Als Quenchflüssigkeit wird beispielsweise eine 40%ige wässrige Isopropanollösung oder n-Butanol verwendet.

### Vergleichsbeispiel 1

Die Länge des Kapillarreaktors betrug 60 m, das Reaktorvolumen betrug 92 ml. Als Einsatzstoffe wurden dem Reaktor 5 Nl/h Propan zugeführt sowie Stickstoffmonoxid und Sauerstoff im Molverhältnis Propan : NO : O₂ = 1 : 1 : 0,5. Der Reaktor wurde auf einem Druck von 12 bar gehalten und auf eine Temperatur von 250°C temperiert. Die Analyse des Produktgemisches ergab eine Selektivität von Propan zu 2-Nitropropan von 54% bei einem Propanumsatz von 49%.

### Vergleichsbeispiel 2

Die Reaktorkonfiguration sowie die Betriebsbedingungen entsprachen denen des Beispiels 1. Allerdings wurde Sauerstoff überstöchiometrisch zugeführt. Das Molverhältnis der Einsatzstoffe war Propan : NO : O₂ = 1 : 1 : 1. Die Analyse des Produktgemisches ergab eine Selektivität von Propan zu 2-Nitropropan von 42% bei einem Propanumsatz von 78%.

### Erfindungsgemäßes Beispiel (O₂-Zwischeneinspeisung)

In weiteren Versuchen wurde das Molverhältnis der Einsatzstoffe erneut geändert zu Propan : NO : O₂ = 1 : 1,2 : 1,2, wobei der Sauerstoff zu gleichen Teilen (3 mal 0,4) am Reaktoreingang, an einer ersten Einspeisestelle bei 20 m Reaktorlänge und an einer zweiten Einspeisestelle bei 40 m Reaktorlänge zugeführt wurde. Diese Fahrweise mit Sauerstoffzwischeneinspeisung wurde für unterschiedliche Temperaturen und Einsatzstoffmengen getestet. Der Druck im Reaktor betrug jeweils 12 bar. Die Ergebnisse sind in der nachfolgenden Tabelle wiedergegeben. Die Selektivität des Propans zu anderen Nitroalkanen (1-Nitropropan, Nitroethan und Nitromethan) in Summe lag für die dargestellten Versuchsergebnisse im Bereich von 9% bis 13%.

| Propanzufuhr [Nl/h] | Temperatur [°C] | Selektivität zu 2-Nitropropan [%] | Propanumsatz [%] |
|---|---|---|---|
| 5 | 250 | 51 | 85 |
| 5 | 270 | 52 | 89 |
| 10 | 250 | 48 | 74 |
| 10 | 270 | 49 | 82 |
| 10 | 290 | 49 | 84 |
| 20 | 270 | 47 | 74 |
| 20 | 290 | 47 | 82 |

In einem kleineren Reaktor wurden die nachfolgend beschriebenen Versuche mit Salpetersäure (HNO₃) und Stickstoffdioxid (NO₂) als Einsatzstoffe durchgeführt:

### Vergleichsbeispiel 3

Für die Nitrierung von Propan mit konzentrierter Salpetersäure (65%) wurden bei einem Molverhältnis von Propan zu HNO₃ von 4 zu 1 ein Strom von 5,6 Nl/h Propan und ein Strom von 6,2 g/h HNO₃ (65%) getrennt kontinuierlich als Edukte in je eine 1/16"-Edelstahlkapillare mit einem kreisrunden Querschnitt und einem Innendurchmesser von 0,76 mm und einer Länge von je 3 m bei einem Druck von 2 bar gefördert. Die Kapillaren befanden sich in einem Ofenraum, der mittels Heißluft auf einer Temperatur von 210°C gehalten wurde. Die Edukte verdampften in den Kapillaren und wurden noch im Ofenraum in einer zweiten Zone bei 250°C über ein T-Stück miteinander vermischt und anschließend in eine Anordnung mit vier parallelen Reaktionskapillaren geleitet, die sich in einem an den ersten Ofenraum anschließenden zweiten Ofenraum befand. Die Reaktionskapillaren wurden mittels Heißluft auf eine Temperatur von 320°C temperiert. Bei jeder der vier parallel angeordneten Reaktionskapillaren handelte es sich um eine 1/16"-Edelstahlkapillare mit einem kreisrunden Querschnitt, einem Innendurchmesser von 0,76 mm und einer Länge von 10 m, die im Inneren mit Silizium beschichtet war. Das gesamte Reaktionsvolumen der vier parallelen Kanäle betrug 18,2 ml. Die Verteilung der Ströme auf die vier Kapillaren und deren anschließende Zusammenführung erfolgte durch jeweils drei T-Stücke derart, dass sich gleiche Strömungswege ergaben, um eine gleichmäßige Durchströmung der vier Kanäle sicherzustellen. Der Druck in den Reaktionskapillaren wurde auf 2 bar gehalten. Die Verweilzeit des Reaktionsgemisches in den Reaktionskapillaren betrug ca. 4 s. Die Analyse des Produktgemischs ergab (Angaben in Fl-%) 97,6% Propan, 1,0% 2-Nitropropan und 0,6% 1-Nitropropan. Unter ansonsten gleichen Versuchsbedingungen wurden für höhere Temperaturen in der Reaktionskapillaren die folgenden Ergebnisse erhalten:
93,3% Propan, 2,5% 2-Nitropropan und 1,7% 1-Nitropropan bei 340°C,
91,1% Propan, 3,3% 2-Nitropropan und 2,6% 1-Nitropropan bei 360°C,
89,8% Propan, 3,2% 2-Nitropropan und 3,2% 1-Nitropropan bei 380°C.

### Vergleichsbeispiel 4

Ein Strom von 9,1 g/h Propan und ein Strom von 9,7 g/h NO₂ wurden getrennt kontinuierlich als Edukte in je eine 1/16"-Edelstahlkapillare mit einem kreisrunden Querschnitt und einem Innendurchmesser von 0,76 mm und einer Länge von je 3 m bei einem Druck von 12 bar gefördert. Die Kapillaren befanden sich in einem Ofenraum, der mittels Umluft auf einer Temperatur von 280°C gehalten wurde. Die Edukte verdampften in den Kapillaren und wurden nach dem Ofenraum in einer zweiten Zone bei 250°C über ein T-Stück miteinander vermischt und anschließend in eine Reaktionskapillare geleitet, die sich in demselben Ofenraum bei einer Temperatur von 280°C befand. Bei der Reaktionskapillare handelte es sich um eine 1/16"-Kapillare mit einem kreisrunden Querschnitt und einem Innendurchmesser von 0,76 mm, einer Länge von 7 m und einem Volumen von 3,5 ml, die im Inneren mit Silizium beschichtet war. Die Verweilzeit des Reaktionsgemisches in der Reaktionskapillare betrug 8 s. Das Ende der Reaktionszone lag außerhalb des Ofenraums in dem auf 250°C temperierten Bereich. Das Produktgemisch wurde dort unverzüglich mit einem Strom von 2,5 g/h Wasserdampf vermischt, über ein Druckhalteventil auf 1 bar entspannt und in einem Doppelmantelrohr auf 20°C abgekühlt. Sowohl die Gasphase als auch der zweiphasige Flüssigaustrag wurden analysiert. Der Propanumsatz betrug 6% bei einer Selektivität des Propans zu Nitroalkanen von 84%. Die Selektivitäten bezüglich der einzelnen Komponenten betrugen 69%, 12%, 1% und 2% für 2-Nitropropan, 1-Nitropropan, Nitroethan und Nitromethan. Als Nebenprodukte wurden 7% Methanol, 8% Aceton und 1% Essigsäure bestimmt.

### Vergleichsbeispiel 5

In demselben Versuchsaufbau wie in Beispiel 1 beschrieben wurden ein Strom von 54 g/h Propan und ein Strom von 10 g/h NO₂ getrennt kontinuierlich als Edukte zugeführt. Der Druck in den Kapillaren betrug 12 bar, der Ofenraum wurde auf 400°C temperiert. Die Zone außerhalb des Ofenraums wies eine Temperatur von 250°C auf. Die Verweilzeit in der Reaktionskapillare betrug 1,9 s. Beim Austritt aus der Reaktionskapillaren wurde das Produktgemisch mit einem Strom von 2,5 g/h Wasserdampf vermischt, über ein Druckhalteventil auf 1 bar entspannt und in einem Doppelmantelrohr auf 20°C abgekühlt. Der zweiphasige Flüssigaustrag wurde analysiert. Bezogen auf die in der Flüssigphase vorhandenen gebildeten organischen Produkte von 0,12 g/h wurden 7% Essigsäure, 28% Nitropropane, 4% Nitroethan und 12% Nitromethan festgestellt.

### Vergleichsbeispiel zu Vergleichsbeispiel 5

Zum Vergleich wurde Beispiel 6 dahingehend abgewandelt, dass eine nicht mit Silizium beschichtete Reaktionskapillare verwendet wurde. Die Eduktströme betrugen 51 g/h Propan und 11 g/h NO₂, der dem Produktgemisch zugesetzte Wasserdampfstrom betrug 5,6 g/h. Druck und Temperaturen waren identisch wie bei Beispiel 6. Die Verweilzeit in der Reaktionskapillaren betrug 2,0 s. Bezogen auf die in der Flüssigphase vorhandenen gebildeten organischen Produkte von 0,1 g/h wurden 20% Essigsäure, 7% Nitropropane, 2% Nitroethan und 14% Nitromethan analysiert.

### Isooktan-Nitrierung

Der Versuchsaufbau zur Synthese von Nitro-Isooktan entspricht im Wesentlichen dem in Fig. 1 dargestellten Reaktorkonzept. Allerdings wird der maximale Systemdruck auf 5 bar begrenzt und der ursprünglich flüssige Einsatzstoff Isooktan in einer Kapillaren bei 200°C verdampft.

### Vergleichsbeispiel 6

Ein Strom von 1 Nl/h NO und ein Strom von 1 Nl/h O₂ wurden separat aus Gasflaschen gasförmig mengengeregelt einem ersten Mischer zugeführt, wobei Stickstoffdioxid (NO₂) entstand. Dieses Gasgemisch wurde als Nitrierungsmittel einem zweiten Mischer zugeleitet. Beide Mischer befanden sich in einem Thermostaten, der auf 200°C eingestellt war. Ein Strom von 2 Nl/h, entsprechend 10,2 g/h, Isooktan wurde in einer 1/16"-Kapillaren derselben Thermostatzone zugeführt. Das Isooktan verdampfte und wurde dem zweiten Mischer zugeführt, wo es sich mit dem Nitrierungsmittel vermischte. Vom zweiten Mischer wurde das Reaktionsgemisch in den Reaktor überführt, der als 1/8"-Kapillarrohr mit einem Innendurchmesser von 1,4 mm ausgestaltet war. Die Innenfläche des Rohres war mit Silizium beschichtet, seine Länge betrug 15 m. Das Kapillarrohr war zylindrisch aufgewickelt und befand sich in einem rohrförmigen Heißluftofen, der fönbeheizt auf 250°C temperiert war. Der Druck in den Zuleitungen, der Thermostatzone und im Reaktor betrug 5 bar.

Der aus dem Reaktor austretende Produktstrom wurde zur Verhinderung einer Produkt-Kondensation auf ca. 200°C gehalten, druckgeregelt auf Umgebungsdruck entspannt und über ein Probenahmeventil zur Analytik geführt. Von der Online-Analytik wurde der Gasstrom weiter in einen Quenchkessel geführt, in dem die flüssigen Reaktionsprodukte drucklos in n-Butanol als Quenchflüssigkeit verdünnt wurden. In der Online-Gasanalytik wurden 69 Fl.-% Isooktan und 8,3 Fl.-% Nitro-Isooktan festgestellt. Der Umsatz an Isooktan betrug 33%, die Selektivität zu Nitro-Isooktan 25%.

### Erfindungsgemäßes Beispiel

Der Versuchsaufbau gemäß Beispiel 6 wurde dahingehend variiert, dass als Reaktor ein 60 m langes 1/8"-Kapillarrohr verwendet wurde. Ferner wurden zwei Zuleitungen für die Zwischeneinspeisung von Sauerstoff vorgesehen, die sich nach einem Drittel und nach zwei Drittel der Reaktorlänge befanden. Der Isooktanstrom betrug 8 Nl/h, entsprechend 40,8 g/h, der NO-Strom betrug 4 Nl/h. Dieser wurde mit einem Strom von 1,33 Nl/h O₂ vermischt, über die Zwischeneinspeisungen wurden nochmals je 1,33 Nl/h O₂ dem Reaktionsgemisch zugefügt, in Summe also ebenfalls 4 Nl/h Sauerstoff. In der Online-Analytik wurden 14,2 Fl.-% Isooktan und 25,3 Fl.-% Nitro-Isooktan ermittelt. Der Umsatz an Isooktan wurde auf 88% gesteigert bei einer Selektivität zu Nitro-Isooktan von 29%.

## Patentansprüche

1. Verfahren zur Herstellung von Nitroalkanen durch Umsetzung mindestens eines Alkans mit mindestens einem Nitrierungsmittel in der Gasphase, **dadurch gekennzeichnet, dass** die Nitrierung in einer mikrostrukturierten Reaktionszone mit parallelen Kanälen mit hydraulischen Durchmessern von weniger als 2,5 mm und einer gesamten spezifischen inneren Oberfläche von mehr als 1600 m²/m³ durchgeführt wird, wobei das Alkan und das Nitrierungsmittel gasförmig unter einem Druck von 1 bar bis 20 bar durch die Reaktionszone gefördert und bei einer Temperatur von 150°C bis 650°C umgesetzt und die Reaktionsprodukte nach der Reaktionszone gekühlt und ausgetragen werden, und wobei als mindestens ein Nitrierungsmittel ein Gemisch aus Stickstoffoxiden und Sauerstoff eingesetzt wird, wobei ein stickstoffoxidreiches Teilgemisch am Reaktoreingang zugeführt wird und ein sauerstoffreiches Teilgemisch über zwei bis zehn Einspeisestellen entlang der Reaktionszone verteilt zugeführt wird.

2. Verfahren nach Anspruch 1, wobei die Nitrierung in Gegenwart einer inerten oder passivierten inneren Oberfläche der mikrostrukturierten Reaktionszone durchgeführt wird.

3. Verfahren nach Anspruch 2, wobei die innere Oberfläche der mikrostrukturierten Reaktionszone mit einer Siliziumbeschichtung versehen ist.

4. Verfahren nach Anspruch 2, wobei die Innenwände der Reaktionskanäle aus einem inerten Material gefertigt sind, insbesondere aus Silizium, Siliziumcarbid oder einem Glas, das bezüglich des Inertverhaltens vergleichbare Eigenschaften wie Quarzglas oder Borosilikatglas aufweist.

5. Verfahren nach einem der Ansprüche 1 bis 4, wobei die Wandstärke zwischen zwei Hohlräumen an jeder Stelle in der mikrostrukturierten Reaktionszone so gewählt ist, dass die Eigensicherheit gegen detonative Explosionen gegeben ist.

6. Verfahren nach einem der Ansprüche 1 bis 5, wobei über die zwei bis zehn Einspeisestellen ein überwiegend Sauerstoff enthaltendes Teilgemisch, insbesondere elementarer Sauerstoff zugeführt wird.

7. Verfahren nach einem der Ansprüche 1 bis 6, wobei dem Reaktionsgemisch zusätzlich inerte Substanzen, insbesondere Stickstoff oder Wasser, zugeführt werden.

8. Verfahren nach einem der Ansprüche 1 bis 7, wobei die Reaktionsprodukte nach Austritt aus der Reaktionszone in einer weiteren mikrostrukturierten Zone um mindestens 10°C, insbesondere um mindestens 30°C abgekühlt werden.

9. Verfahren nach einem der Ansprüche 1 bis 8 zur Herstellung von Nitropropanen durch Umsetzung von Propan mit einem Nitrierungsmittel bei einem Druck von 1 bar bis 20 bar und einer Temperatur von 200°C bis 350°C.

10. Verfahren nach einem der Ansprüche 1 bis 8 zur Herstellung von Nitro-Isooktan durch Umsetzung von Isooktan mit einem Nitrierungsmittel bei einem Druck von 1 bar bis 20 bar und einer Temperatur von 200°C bis 350°C.

## Claims

1. A process for preparing nitroalkanes by reaction of at least one alkane with at least one nitrating agent in the gas phase, wherein the nitration is carried out in a microstructured reaction zone having parallel channels having hydraulic diameters of less than 2.5 mm and a total specific internal surface area of more than 1600 m²/m³, where the alkane and the nitrating agent are conveyed in gaseous form under a pressure of from 1 bar to 20 bar through the reaction zone and reacted at a temperature of from 150°C to 650°C and the reaction products are cooled downstream of the reaction zone and discharged and the at least one nitrating agent used is a mixture of nitrogen oxides and oxygen, where a nitrogen oxide-rich partial mixture is introduced at the reactor inlet and an oxygen-rich partial mixture is introduced via from two to ten introduction points along the reaction zone.

2. The process according to claim 1, wherein the nitration is carried out in the presence of an inert or passivated internal surface of the microstructured reaction zone.

3. The process according to claim 2, wherein the internal surface of the microstructured reaction zone is provided with a silicon coating.

4. The process according to claim 2, wherein the interior walls of the reaction channels are made of an inert material, in particular silicon, silicon carbide or a glass which in respect of inertness has comparable properties to fused silica or borosilicate glass.

5. The process according to any of claims 1 to 4, wherein the wall thickness between two hollow spaces at every place in the microstructured reaction zone is selected so that intrinsic safety in respect of detonative explosions is ensured.

6. The process according to any of claims 1 to 5, wherein a partial mixture comprising predominantly oxygen, in particular elemental oxygen, is introduced via the from two to ten introduction points.

7. The process according to any of claims 1 to 6, wherein inert substances, in particular nitrogen or water, are additionally added to the reaction mixture.

8. The process according to any of claims 1 to 7, wherein the reaction products are cooled by at least 10°C, in particular by at least 30°C, in a further microstructured zone after exit from the reaction zone.

9. The process according to any of claims 1 to 8 for preparing nitropropanes by reaction of propane with a nitrating agent at a pressure of from 1 bar to 20 bar and a temperature of from 200°C to 350°C.

10. The process according to any of claims 1 to 8 for preparing nitroisooctane by reaction of isooctane with a nitrating agent at a pressure of from 1 bar to 20 bar and a temperature of from 200°C to 350°C.

## Revendications

1. Procédé pour la préparation de nitroalcanes par transformation d'au moins un alcane avec au moins un agent de nitration en phase gazeuse, **caractérisé en ce que** la nitration est réalisée dans une zone de réaction microstructurée pourvue de canaux parallèles présentant des diamètres hydrauliques inférieurs à 2,5 mm et une surface interne spécifique totale supérieure à 1600 m²/m³, l'alcane et l'agent de nitration étant transportés sous forme gazeuse à une pression de 1 bar à 20 bars à travers la zone de réaction et transformés à une température de 150°C à 650°C et les produits de réaction étant refroidis après la zone de réaction et évacués et un mélange d'oxydes d'azote et d'oxygène étant utilisé comme au moins un agent de nitration, un mélange partiel riche en oxydes d'azote étant introduit au niveau de l'entrée du réacteur et un mélange partiel riche en oxygène étant introduit de manière répartie sur deux à dix sites d'injection le long de la zone de réaction.

2. Procédé selon la revendication 1, la nitration étant réalisée en présence d'une surface interne inerte ou passivée de la zone de réaction microstructurée.

3. Procédé selon la revendication 2, la surface interne de la zone de réaction microstructurée étant pourvue d'un revêtement à base de silicium.

4. Procédé selon la revendication 2, les parois internes des canaux de réaction étant fabriquées en un matériau inerte, en particulier en silicium, en carbure de silicium ou en un verre, qui présente, en ce qui concerne le comportement inerte, des propriétés comparables à celles du verre à quartz ou du verre borosilicaté.

5. Procédé selon l'une quelconque des revendications 1 à 4, l'épaisseur de paroi entre deux espaces creux en chaque endroit de la zone de réaction microstructurée étant choisie de manière telle que la sécurité autonome contre des explosions avec détonation est assurée.

6. Procédé selon l'une quelconque des revendications 1 à 5, un mélange partiel contenant principalement de l'oxygène, en particulier de l'oxygène élémentaire, étant introduit via lesdits deux à dix sites d'injection.

7. Procédé selon l'une quelconque des revendications 1 à 6, le mélange réactionnel étant en outre additionné de substances inertes, en particulier d'azote ou d'eau.

8. Procédé selon l'une quelconque des revendications 1 à 7, les produits de réaction étant refroidis d'au moins 10°C, en particulier d'au moins 30°C, après la sortie de la zone de réaction, dans une autre zone microstructurée.

9. Procédé selon l'une quelconque des revendications 1 à 8 pour la préparation de nitropropanes par transformation de propane avec un agent de nitration à une pression de 1 bar à 20 bars et à une température de 200°C à 350°C.

10. Procédé selon l'une quelconque des revendications 1 à 8 pour la préparation de nitro-iso-octane par transformation d'iso-octane avec un agent de nitration à une pression de 1 bar à 20 bars et à une température de 200°C à 350°C.
